# EUROPEAN PATENT APPLICATION

(11) **EP 4 293 107 A1**
(43) Date of publication of application: **20.12.2023**
(21) Application number: 22750046.9
(22) Date of filing: 04.02.2022
(51) Int. Cl.: C12N 5/071

(54) **METHOD FOR DIFFERENTIATING PLURIPOTENT STEM CELL-DERIVED HEMOGENIC ENDOTHELIAL CELLS INTO LYMPHOID LINEAGE CELLS**

(30) Priority: 05.02.2021 KR 20210016845
(71) Applicant: Sungkwang Medical Foundation, Seoul 06135 (KR)
(72) Inventor: LEE, Ji Yoon, Seongnam-si, Gyeonggi-do 13488 (KR); LEE, Ah Reum, Seongnam-si, Gyeonggi-do 13488 (KR); PARK, Jung Eun, Gimpo-si, Gyeonggi-do 10068 (KR)
(74) Representative: Weickmann & Weickmann PartmbB
(86) International application number: PCT/KR2022/001757
(87) International publication number: WO 2022/169297

(57) **Abstract**

Provided is a method of differentiating a pluripotent stem cell-derived hemogenic endothelial cell into a lymphoid lineage blood cell, enabling a hemogenic endothelial cell to be cultured for a long period of time so as to be induced into a lymphoid lineage blood cell, thereby facilitating induction of generation of immune cells such as T cells, B cells, and NK cells.

## Description

### Technical Field

This application is based on and claims priority under 35 U.S.C. §119 to Korean Patent Application No. 10-2021-0016845, filed on February 5, 2021, in the Korean Intellectual Property Office, the disclosure of which is incorporated by reference herein in its entirety.

The present application relates to a method of differentiating pluripotent stem cell-derived hemogenic endothelial cells into lymphoid lineage blood cells.

### Background Art

The generation and maintenance of all blood vessels rely on a very small number of hematopoietic stem cells present in the bone marrow of adults. Transplantation of hematopoietic stem cells derived from bone marrow, mobilized peripheral blood, or umbilical cord blood (UCB) is used as a standard for treating various genetic or malignant diseases. However, the limited potential of human leukocyte antigen (HLA)-matched donors remains a major challenge, and despite antigen incompatibility, the nature of UCB is such that a relatively small number of hematopoietic stem cells can delay engraftment or cause various problems in engraftment after transplantation.

Meanwhile, JP 5995237 discloses a method for differentiation of animal aorta-gonad-mesonephor (AGM)-derived angioblasts into human into human angioblast, and US 2004-0235160 discloses a process for effectively generating hematopoietic stem cells in animals. As such, the generation of angioblasts and the development of differentiation methods for blood cells using the angioblasts are becoming increasingly important. However, in fact, there are few reports on a culture technique for vascular cell differentiation that differentiates human angioblasts directly into blood cells, and there is no method that can induce differentiation into lymphoid lineage blood cells. Therefore, there is a need to establish an optimized long-term culture method for angioblasts that enables the generation of blood cells.

### Disclosure

### Technical Problem

An aspect is to provide a method of preparing a lymphoid lineage blood cell from a pluripotent stem cell, the method including: differentiating a pluripotent stem cell into a mesodermal cell by culturing in a first medium containing bFGF, VEGF, and SCF; differentiating the mesodermal cell into an early hemogenic endothelial cell (EHE) by culturing in a second medium containing TPO, EPO, and IGF-1; and differentiation of the EHE cell into a late hemogenic endothelial cell (LHE) by culturing in a third medium containing IL-5, IL-7, and DLL.

### Technical Solution

An aspect provides a method of preparing a lymphoid lineage blood cell from a pluripotent stem cell, the method including: differentiating a pluripotent stem cell into a mesodermal cell by culturing in a first medium containing a first medium composition containing bFGF, VEGF, and SCF; differentiating the mesodermal cell into an early hemogenic endothelial cell (EHE) by culturing in a second medium containing a second medium composition containing TPO, EPO, and IGF-1; and differentiation of the EHE cell into a late hemogenic endothelial cell (LHE) by culturing in a third medium containing a third medium composition containing IL-5, IL-7, and DLL.

The term "pluripotent stem cells" as used in the present specification refers to self-renewing stem cells that are pluripotent or totipotent, capable of differentiating into cells of all tissues of an individual. The pluripotent stem cells may include, for example, embryonic stem cells, induced pluripotent stem cells (iPSC), somatic cell nuclear transfer-derived stem cells, and the like.

The term "hemogenic endothelial cells" as used in the present specification refers to differentiated rare vascular endothelial cells, which can differentiate into hematoblasts during embryogenesis. The development of hematoblasts in the embryo proceeds from the mesoderm sequentially to hemangioendothelial precursor cells and hemopoietic precursor cells through angioblasts.

The term "angioblasts (or vasoformative cells)" as used in the present specification refers to a kind of endothelial precursor cells derived from bone marrow, and is one of mesenchymal cells capable of developing into endothelium of blood vessels.

The term "lymphoid lineage blood cells" as used in the present specification refers to lineage cells differentiated from hematopoietic stem cells, and is mainly involved in adaptive immunity and innate immunity. The lymphoid lineage blood cells may include, for example, natural killer cells (NK cells), T lymphocytes, and B lymphocytes. Thus, the method according to an aspect can efficiently differentiate pluripotent stem cells into lymphoid lineage blood cells such as NK cells, T lymphocytes, or B lymphocytes, *in vitro.*

The method according to an embodiment includes differentiating pluripotent stem cells into mesodermal cells by culturing in a first medium containing a first medium composition containing bFGF, VEGFA and SCF. The medium composition may be included in a common cell culture medium. The cell culture medium may be, for example, Dulbecco's Modified Eagle's Medium (DMEM, GIBCO, USA), Minimal Essential Medium (MEM, GIBCO, USA), basic medium Eagle (BME, GIBCO, USA), RPMI 1640 (GIBCO, USA) ), Dulbecco's Modified Eagle's Medium: Nutrient Mixture F-10 (DMEM/F10, GIBCO, USA), Dulbecco's Modified Eagle's Medium: Nutrient Mixture F-12 (DMEM/F12, GIBCO, USA), α-Minimal essential Medium (α-MEM, GIBCO, USA), Glasgow's Minimal Essential Medium (G-MEM, GIBCO, USA), Isocove's Modified Dulbecco's Medium (IMDM, GIBCO, USA), STEMdiff APEL 2 Medium (Apel II), Endothelial Cell Growth medium-2 (EGM-2), and the like.

The bFGF may be included at a concentration in a range of 1 ng/ml to 25 ng/ml. The bFGF may be, for example, included at a concentration in a range of 1 mg/ml to 25 ng/ml, 1 mg/ml to 20 ng/ml, 1 mg/ml to 15 ng/ml, 5 mg/ml to 25 ng/ml, 5 mg/ml to 20 ng/ml, 5 mg/ml to 15 ng/ml, or 7 mg/ml to 12 ng/ml. When the content of bFGF exceeds the ranges above, there is a problem that a differentiation rate of the pluripotent stem cells into mesodermal cells is slow or that differentiated cells tend to show characteristics similar to embryonic stem cells.

In addition, the VEGFA may be included at a concentration in a range of 5 ng/ml to 40 ng/ml. The VEGFA may be, for example, included at a concentration in a range of 5 ng/ml to 40 ng/ml, 5 ng/ml to 35 ng/ml, 5 ng/ml to 30 ng/ml, 5 ng/ml to 25 ng/ml, 5 ng/ml to 20 ng/ml, 10 ng/ml to 40 ng/ml, 10 ng/ml to 35 ng/ml, 10 ng/ml to 30 ng/ml, or 15 ng/ml to 25 ng/ml. When the content of VEGFA is less than the ranges above, there is a problem that cell proliferation is not smooth, and when the content of VEGFA exceeds the ranges above, there is a problem that pluripotent stem cells may be differentiated into vascular endothelial cells.

In addition, the SCF may be included at a concentration in a range of 20 ng/ml to 70 ng/ml. The SGF may be, for example, included at a concentration in a range of 20 ng/ml to 70 ng/ml, 20 ng/ml to 65 ng/ml, 20 ng/ml to 60 ng/ml, 20 ng/ml to 55 ng/ml, 25 ng/ml to 70 ng/ml, 25 ng/ml to 65 ng/ml, 25 ng/ml to 60 ng/ml, 25 ng/ml to 55 ng/ml, 30 ng/ml to 70 ng/ml, 30 ng/ml to 65 ng/ml, or 40 ng/ml to 60 ng/ml.

In an embodiment, the first composition may further include ascorbic acid, BMP4, CHIR, or a mixture thereof. The ascorbic acid may be included at a concentration in a range of 50 ng/ml to 250 ng/ml. The ascorbic acid may be, for example, included at a concentration in a range of 50 ng/ml to 250 ng/ml, 50 ng/ml to 200 ng/ml, 50 ng/ml to 150 ng/ml, 70 ng/ml to 250 ng/ml, 70 ng/ml to 230 ng/ml, 70 ng/ml to 150 ng/ml, or 80 ng/ml to 150 ng/ml.

In addition, the BMP4 may be included at a concentration in a range of 10 ng/ml to 50 ng/ml. The BMP4 may, for example, included at a concentration in a range of 10 ng/ml to 50 ng/ml, 10 ng/ml to 45 ng/ml, 10 ng/ml to 40 ng/ml, 10 ng/ml to 35 ng/ml, 10 ng/ml to 30 ng/ml, 15 ng/ml to 50 ng/ml, 15 ng/ml to 45 ng/ml, 15 ng/ml to 40 ng/ml, or 20 ng/ml to 35 ng/ml.

In addition, the CHIR may be included at a concentration in a range of 0.5 nM to 10 nM. The CHIR may be, for example, included at a concentration in a range of 0.5 nM to 10 nM, 1 nM to 10 nM, 1 nM to 8 nM, 1 nM to 6 nM, 1 nM to 4 nM, 2 nM to 9 nM, 2 nM to 7 nM, 2 nM to 5 nM, or 3 nM to 5 nM. When the content of CHIR is less than the ranges above, there is a problem that differentiation of pluripotent stem cells into mesodermal cells is not smooth, and when the content of CHIR exceeds the ranges above, there is a problem that pluripotent stem cells may be differentiated into hepatocytes.

In an embodiment, the differentiating of the pluripotent stem cells into mesodermal cells may be performed for 1 day to 3 days. For example, the differentiating of the pluripotent stem cells into mesodermal cells may be performed for 1 day to 3 days, 1 day to 2 days, or 2 days to 3 days. When the culture period is less than the ranges above, there is a problem that the pluripotent stem cells may not be sufficiently differentiated into mesodermal cells, and when the culture period exceeds the ranges above, there is a problem that the pluripotent stem cells may not be differentiated into hemogenic endothelial cells in mesoderm and may be differentiated into other lineage blood cells.

The method according to an embodiment includes differentiating the mesodermal cells into early hemogenic endothelial cells (EHE cells) in a second medium in which a second medium composition containing TPO, EPO and IGF-1 is included in a basic medium.

The TPO may be included at a concentration in a range of 50 ng/ml to 250 ng/ml. The TPO may be, for example, included at a concentration in a range of 50 ng/ml to 250 ng/ml, 50 ng/ml to 200 ng/ml, 50 ng/ml to 150 ng/ml, 70 ng/ml to 250 ng/ml, 70 ng/ml to 230 ng/ml, 70 ng/ml to 150 ng/ml, or 80 ng/ml to 150 ng/ml. When the content of TPO is less than the ranges above, there is a problem that a definitive hematopoiesis process is not smooth, and when the content of TPO exceeds the ranges above, there is a problem that intensive differentiation into megakaryocytes may occur.

In addition, the EPO may be included at a concentration in a range of 5 ng/ml to 40 ng/ml. The EPO may be, for example, included at a concentration in a range of 5 ng/ml to 40 ng/ml, 5 ng/ml to 35 ng/ml, 5 ng/ml to 30 ng/ml, 5 ng/ml to 25 ng/ml, 5 ng/ml to 20 ng/ml, 10 ng/ml to 40 ng/ml, 10 ng/ml to 35 ng/ml, 10 ng/ml to 30 ng/ml, or 15 ng/ml to 25 ng/ml. When the content of EPO is less than the ranges above, there is a problem that a long time is required to generate red blood cells.

In addition, the IGF-1 may be included at a concentration in a range of 20 ng/ml to 70 ng/ml. The IGF-1 may be, for example, included at a concentration in a range of 20 ng/ml to 70 ng/ml, 20 ng/ml to 65 ng/ml, 20 ng/ml to 60 ng/ml, 20 ng/ml to 55 ng/ml, 25 ng/ml to 70 ng/ml, 25 ng/ml to 65 ng/ml, 25 ng/ml to 60 ng/ml, 25 ng/ml to 55 ng/ml, 30 ng/ml to 70 ng/ml, 30 ng/ml to 65 ng/ml, or 40 ng/ml to 60 ng/ml. When the content of IGF-1 is less than the ranges above, there is a problem that a blood differentiation commitment step is not smooth, and when the content of IGF-1 exceeds the ranges above, a blood differentiation commitment step is limited to myeloid lineage cells.

In an embodiment, the second composition may further include bFGF, VEGFA, SCF, FLT3L, GCSF, and IL-6, or a mixture thereof.

The bFGF may be included at a concentration in a range of 1 ng/ml to 25 ng/ml. The bFGF may be, for example, included at a concentration in a range of 1 mg/ml to 25 ng/ml, 1 mg/ml to 20 ng/ml, 1 mg/ml to 15 ng/ml, 5 mg/ml to 25 ng/ml, 5 mg/ml to 20 ng/ml, 5 mg/ml to 15 ng/ml, or 7 mg/ml to 12 ng/ml.

In addition, the VEGFA may be included at a concentration in a range of 50 ng/ml to 250 ng/ml. The VEGFA may be, for example, included at a concentration in a range of 50 ng/ml to 250 ng/ml, 50 ng/ml to 200 ng/ml, 50 ng/ml to 150 ng/ml, 70 ng/ml to 250 ng/ml, 70 ng/ml to 230 ng/ml, 70 ng/ml to 150 ng/ml, or 80 ng/ml to 150 ng/ml.

In addition, the SCF may be included at a concentration in a range of 100 ng/ml to 500 ng/ml. The SCF may be, for example, included at a concentration in a range of 100 ng/ml to 500 ng/ml, 100 ng/ml to 450 ng/ml, 100 ng/ml to 400 ng/ml, 100 ng/ml to 350 ng/ml, 150 ng/ml to 500 ng/ml, 150 ng/ml to 450 ng/ml, 150 ng/ml to 350 ng/ml, 200 ng/ml to 500 ng/ml, 250 ng/ml to 400 ng/ml, 170 ng/ml to 330 ng/ml, or 220 ng/ml to 300 ng/ml.

In addition, the FLT3L may be included at a concentration in a range of 100 ng/ml to 400 ng/ml. The FLT3L may be, for example, included at a concentration in a range of 100 ng/ml to 400 ng/ml, 100 ng/ml to 350 ng/ml, 100 ng/ml to 320 ng/ml, 100 ng/ml to 300 ng/ml, 100 ng/ml to 280 ng/ml, 100 ng/ml to 260 ng/ml, 100 ng/ml to 240 ng/ml, 100 ng/ml to 220 ng/ml, 150 ng/ml to 400 ng/ml, 150 ng/ml to 300 ng/ml, or 150 ng/ml to 250 ng/ml. When the content of FLT3L is less than the ranges above, there is a problem that the concentration of differentiation of the EHE cells into blood cells may decrease.

In addition, the GCSF may be included at a concentration in a range of 5 ng/ml to 50 ng/ml. The GCSF may be, for example, included at a concentration in a range of 5 ng/ml to 50 ng/ml, 5 ng/ml to 45 ng/ml, 5 ng/ml to 40 ng/ml, 5 ng/ml to 35 ng/ml, 5 ng/ml to 30 ng/ml, 5 ng/ml to 25 ng/ml, 10 ng/ml to 50 ng/ml, 10 ng/ml to 45 ng/ml, 10 ng/ml to 40 ng/ml, 10 ng/ml to 35 ng/ml, 15 ng/ml to 30 ng/ml, or 15 ng/ml to 25 ng/ml. When the content of GCSF is less than the ranges above, the differentiation of EHE cells into myeloid lineage cells may not be promoted well.

In addition, the IL-6 may be included at a concentration in a range of 10 ng/ml to 70 ng/ml. The IL-6 may be, for example, include at a concentration in a range of 10 ng/ml to 70 ng/ml, 10 ng/ml to 65 ng/ml, 10 ng/ml to 60 ng/ml, 10 ng/ml to 55 ng/ml, 20 ng/ml to 70 ng/ml, 20 ng/ml to 65 ng/ml, 20 ng/ml to 60 ng/ml, 30 ng/ml to 60 ng/ml, or 45 ng/ml to 55 ng/ml. When the content of IL-6 is less than the ranges above, definitive lymphopoiesis may not be achieved well.

In an embodiment, the differentiating of the mesodermal cells into EHE cells may be performed for 6 day to 8 days. When the culture period is less than the range above, there is a problem that the growth and expansion of hemogenic endothelial cells may be stunted, and when the culture period exceeds the range above, there is a problem that the hemogenic endothelial cells are not sufficiently differentiated from the mesoderm so that the frequency of hemogenic endothelial cells is reduced.

The EHE cells may have one or more characteristics selected from the following (a) to (d):
(a) having rod-type morphological characteristics;
(b) having bright yellow light;
(c) having a doubling time in a range of 15 hours to 35 hours; and
(d) having surface antigen characteristics of CD31+, Tie-2+, CD44+, CD34+, or a combination thereof.

The method includes differentiating the EHE cells into late hemogenic endothelial cells (LHE cells) by culturing in a third medium containing a third medium composition containing IL-5, IL-7, and DLL.

The IL-5 may be included at a concentration in a range of 20 ng/ml to 70 ng/ml. The IL-5 may be, for example, included at a concentration in a range of 20 ng/ml to 70 ng/ml, 20 ng/ml to 65 ng/ml, 20 ng/ml to 60 ng/ml, 20 ng/ml to 55 ng/ml, 25 ng/ml to 70 ng/ml, 25 ng/ml to 65 ng/ml, 25 ng/ml to 60 ng/ml, 30 ng/ml to 60 ng/ml, or 45 ng/ml to 55 ng/ml. When the content of IL-5 is less than the ranges above, there is a problem that the number of lymphoid lineage blood cells may be small, and when the content of IL-5 exceeds the ranges above, there is a problem that red blood cells are generated in large quantities.

In addition, the IL-7 may be included at a concentration in a range of 5 ng/ml to 40 ng/ml. The IL-7 may be, for example, included at a concentration in a range of 5 ng/ml to 40 ng/ml, 5 ng/ml to 35 ng/ml, 5 ng/ml to 30 ng/ml, 5 ng/ml to 25 ng/ml, 10 ng/ml to 40 ng/ml, 10 ng/ml to 35 ng/ml, 10 ng/ml to 30 ng/ml, or 15 ng/ml to 35 ng/ml. When the content of IL-7 is less than the ranges above, differentiation into NK cells may not be smooth.

In addition, the DDL1 may be included at a concentration in a range of 5 ng/ml to 50 ng/ml. The DDL1 may be, for example, included at a concentration in a range of 5 ng/ml to 50 ng/ml, 5 ng/ml to 45 ng/ml, 5 ng/ml to 40 ng/ml, 5 ng/ml to 35 ng/ml, 5 ng/ml to 30 ng/ml, 10 ng/ml to 50 ng/ml, 10 ng/ml to 45 ng/ml, 10 ng/ml to 40 ng/ml, 10 ng/ml to 30 ng/ml, 15 ng/ml to 30 ng/ml, or 20 ng/ml to 30 ng/ml. When the content of DDL1 is less than the ranges above, there is a problem that the proliferation rate of lymphoid lineage blood cells among blood cells is reduced or the lymphoid lineage blood cells are not well separated from angioblasts, and when the content of DDL exceeds the ranges above, differentiation into T cells may be easier than NK cells.

In an embodiment, the third medium may further include bFGF, VEGFA, SCF, FLT3L, GCSF, and IL-6, IGF-1, IL-15, or a mixture thereof.

The bFGF may be included at a concentration in a range of 1 ng/ml to 25 ng/ml. The bFGF may be, for example, included at a concentration in a range of 1 mg/ml to 25 ng/ml, 1 mg/ml to 20 ng/ml, 1 mg/ml to 15 ng/ml, 5 mg/ml to 25 ng/ml, 5 mg/ml to 20 ng/ml, 5 mg/ml to 15 ng/ml, or 7 mg/ml to 12 ng/ml.

In addition, the VEGFA may be included at a concentration in a range of 5 ng/ml to 250 ng/ml. The VEGFA may be, for example, included at a concentration in a range of 50 ng/ml to 250 ng/ml, 50 ng/ml to 200 ng/ml, 50 ng/ml to 150 ng/ml, 70 ng/ml to 250 ng/ml, 70 ng/ml to 230 ng/ml, 70 ng/ml to 150 ng/ml, or 80 ng/ml to 150 ng/ml.

In addition, the SCF may be included at a concentration in a range of 100 ng/ml to 500 ng/ml. The SCF may be, for example, included at a concentration in a range of 100 ng/ml to 500 ng/ml, 100 ng/ml to 450 ng/ml, 100 ng/ml to 350 ng/ml, 100 ng/ml to 250 ng/ml, 100 ng/ml to 150 ng/ml, 150 ng/ml to 500 ng/ml, 150 ng/ml to 400 ng/ml, 150 ng/ml to 300 ng/ml, 170 ng/ml to 250 ng/ml, 200 ng/ml to 250 ng/ml, or 300 ng/ml to 350 ng/ml.

In addition, the FLT3L may be included at a concentration in a range of 100 ng/ml to 400 ng/ml. The FLT3L may be, for example, included at a concentration in a range of 100 ng/ml to 400 ng/ml, 100 ng/ml to 350 ng/ml, 100 ng/ml to 320 ng/ml, 100 ng/ml to 300 ng/ml, 100 ng/ml to 280 ng/ml, 100 ng/ml to 260 ng/ml, 100 ng/ml to 240 ng/ml, 100 ng/ml to 220 ng/ml, 150 ng/ml to 400 ng/ml, 150 ng/ml to 300 ng/ml, or 150 ng/ml to 250 ng/ml.

In addition, the GCSF may be included at a concentration in a range of 5 ng/ml to 50 ng/ml. The GCSF may be, for example, included at a concentration in a range of 5 ng/ml to 50 ng/ml, 5 ng/ml to 45 ng/ml, 5 ng/ml to 40 ng/ml, 5 ng/ml to 35 ng/ml, 5 ng/ml to 30 ng/ml, 5 ng/ml to 25 ng/ml, 10 ng/ml to 50 ng/ml, 10 ng/ml to 45 ng/ml, 10 ng/ml to 40 ng/ml, 10 ng/ml to 35 ng/ml, 15 ng/ml to 30 ng/ml, or 15 ng/ml to 25 ng/ml.

In addition, the IL-6 may be included at a concentration in a range of 10 ng/ml to 70 ng/ml. The IL-6 may be, for example, include at a concentration in a range of 10 ng/ml to 70 ng/ml, 10 ng/ml to 65 ng/ml, 10 ng/ml to 60 ng/ml, 10 ng/ml to 55 ng/ml, 20 ng/ml to 70 ng/ml, 20 ng/ml to 65 ng/ml, 20 ng/ml to 60 ng/ml, 30 ng/ml to 60 ng/ml, or 45 ng/ml to 55 ng/ml.

In addition, the IGF-1 may be included at a concentration in a range of 20 ng/ml to 70 ng/ml. The IGF-1 may be, for example, included at a concentration in a range of 20 ng/ml to 70 ng/ml, 20 ng/ml to 65 ng/ml, 20 ng/ml to 60 ng/ml, 20 ng/ml to 55 ng/ml, 25 ng/ml to 70 ng/ml, 25 ng/ml to 65 ng/ml, 25 ng/ml to 60 ng/ml, 25 ng/ml to 55 ng/ml, 30 ng/ml to 70 ng/ml, 30 ng/ml to 65 ng/ml, or 40 ng/ml to 60 ng/ml.

In addition, the IL-15 may be included at a concentration in a range of 5 ng/ml to 40 ng/ml. The IL-15 may be, for example, included at a concentration in a range of 5 ng/ml to 40 ng/ml, 5 ng/ml to 35 ng/ml, 5 ng/ml to 30 ng/ml, 5 ng/ml to 25 ng/ml, 5 ng/ml to 20 ng/ml, 10 ng/ml to 40 ng/ml, 10 ng/ml to 35 ng/ml, 10 ng/ml to 30 ng/ml, or 15 ng/ml to 25 ng/ml. When the content of IL-15 is less than the ranges above, there is a difficulty in differentiating into and maintaining NK cells, and when the content of IL-15 exceeds the ranges above, encouragement of differentiation into other cells such as T cells also occurs so that it becomes difficult to target only or selectively differentiate into only one of lymphoid lineage blood cells.

In an embodiment, the differentiating of the EHE cells into LHE cells may be performed for 12 day to 13 days. When the differentiation period is less than the range above, there is a problem that the primary hematopoiesis process does not sufficiently occur, which affects the cell generation and frequency thereof in the definitive hematopoiesis process to be reduced.

The LHE cells may have one or more characteristics selected from the following (a) and (b):
(a) having cobblestone-like type morphological characteristics; and
(b) having surface antigen characteristics of CD31+, CD34+, CD144+, Flk-1+, CD144+CD31+, CD144+CD34+, CD31+CD34+, Flk-1+CD34+, or a combination thereof.

The method may further include differentiating the LHE cells into lymphoid lineage blood cells by additionally culturing the LHE cells for 13 days to 28 days. For example, the LHE cells may be additionally cultured for 13 days to 28 days, 13 days to 27 days, 13 days to 26 days, 13 days to 25 days, 13 days to 24 days, 13 days to 22 days, 13 days to 20 days, 15 days to 28 days, 15 days to 25 days, or 20 days to 28 days. When the culture period is less than the ranges above, there is a problem that the hemogenic endothelial cells are not sufficiently differentiated into lymphoid lineage blood cells, and when the culture period exceeds the ranges above, the promotion of lymphoid lineage blood cells is delayed, which fails to sufficiently obtain lymphoid lineage blood cells.

In addition, the differentiating the LHE cells into lymphoid lineage blood cells may be culturing the LHE cells in an EGM-2 basic medium containing a medium composition additionally containing at least one selected from the group consisting of bFGF, VEGFA, SCF, FLT3L, GCSF, IL-6, IGF-1, IL-7, IL-15, IL-5, and DLL1.

That is, the method according to an aspect may enable long-term culture of hemogenic endothelial cells by optimizing medium conditions for the differentiation suitability of lymphoid lineage blood cells and applying the medium at each differentiation step.

Another aspect provides a lymphoid lineage blood cell prepared by the method.

Another aspect provides a pharmaceutical composition for preventing or treating a cancer, including the lymphoid lineage blood cell as an active ingredient. Another aspect provides a cell therapy product including the lymphoid lineage blood cell as an active ingredient. Another aspect provides a method of preventing or treating cancer, the method including administering a lymphoid lineage blood cell of the active ingredient, a cell population thereof, or a culture medium thereof, to a subject in need thereof.

Specific details of the lymphoid lineage blood cell are as described above. The lymphoid lineage blood cell prepared by the method has immune activity, and thus can be used as a cancer immunotherapy product.

### Advantageous Effects

The method according to an aspect enables long-term culture of hemogenic endothelial cells and accordingly induction into lymphoid lineage blood cells, and thus can facilitates the generation of immune cells such as T cells, B cells, and NK cells.

### Description of Drawings

FIG. 1 shows characteristics of early and late hemogenic endothelial cells.
FIG. 2A shows photographs depicting the cell morphology of late hematopoietic endothelial cells according to culture time and shows a graph of proliferation of single cells.
FIG. 2B shows photographs confirming expression of marker proteins in late hemogenic endothelial cells.
FIG. 3A shows photographs depicting cell morphology on Day 1, Day 3, Day 7 and beyond, after culturing hemogenic endothelial (HE) cells in Apel II basic medium.
FIG. 3B shows photographs depicting cell morphology on Day 1 and Day 3, after culturing HE in EGM-2 basic medium.
FIG. 3C shows photographs depicting cell morphology on Day 1, Day 3, Day 7 and beyond, after culturing HE in DMEM/F12 basic medium.
FIG. 4A shows results of confirming expression of marker proteins in late hemogenic endothelial cells.
FIG. 4B shows photographs of confirming Tie-2 enrichment of CD31 cells.
FIG. 5A shows results of confirming generation of lymphoid lineage blood cells from hemogenic endothelial cells in the liver of mice.
FIG. 5B shows results of confirming generation of lymphoid lineage blood cells from hemogenic endothelial cells in the AGM of mice.
FIG. 5C shows a chart summarizing properties and colony formation of hemogenic endothelial cells cultured in each basic medium and the degree of generation of lymphoid lineage blood cells and myeloid lineage cells, and shows results of confirming the shape of hemogenic endothelial cells.
FIG. 5D shows a graph of results of analyzing expression levels of CD45, CD4, CD8, and NK1.1 by using differentiated cells in AGM.
FIG. 6A shows results of cell properties and expression markers at each generation stage of lymphoid lineage blood cells from hemogenic endothelial cells derived from human pluripotent stem cells.
FIG. 6B shows a graph confirming, by expression of lymphoid lineage transcription factors, whether or not differentiation into lymphoid lineage blood cells is achieved in spots treated with IL-5 and DDL1.
FIG. 7A shows results of confirming surface markers of γδT cells, which are progenitor cells of T lymphocyte, by FACS on Day 31 of culture of late hemogenic endothelial cells derived from human pluripotent stem cells.
FIG. 7B shows graphs of quantification of surface markers of γδT cells, which are progenitor cells of T lymphocyte, on Day 31 of culture of late hemogenic endothelial cells derived from human pluripotent stem cells.
FIG. 7C shows results of confirming shape of γδT cells, which are progenitor cells of T lymphocyte, on Day 31 of culture of late hemogenic endothelial cells derived from human pluripotent stem cells.

### BEST MODE

### Mode for Invention

Hereinafter, preferable Examples are presented to help understanding of the present disclosure. However, Examples below are only presented for easier understanding of the present disclosure, and the contents of the present disclosure are not limited by the following examples.

### [Preparation Examples]

### Preparation Example 1. Establishment of culture conditions for differentiation and maintenance of stem cells into hemogenic endothelial cells

Conditions for differentiation and culture of stem cells into hemogenic endothelial cells were confirmed. Specifically, 2.0 x 10⁵ stem cells (hereinafter referred to as 'CHA52 cells') obtained from CHA Biotech Co., Ltd. were seeded onto a single well treated with Matrigel of a 6-well culture dish and cultured for two days. Afterwards, the cells were cultured for 3 days in a mesoderm-specific conditioned medium in which 7 ng/ml of bFGF, 1 nM of CHIR, 60 ng/ml of ascorbic acid, 15 ng/ml of VEGFA, 35 ng/ml of SCF, and 18 ng/ml of BMP4 were added in a basic medium (Stemline II medium) (hereinafter, 'Step I'). Then, the cells were additionally cultured for 6 days in an optimized medium in which 7 ng/ml of bFGF, 15 ng/ml of VEGFA, 180 ng/ml of SCF, 150 ng/ml of FLT3L, 18 ng/ml of GCSF, 80 ng/ml of TPO, 17 ng/ml of EPO, 25 ng/ml of IL-6, and 30 ng/ml of IGF-1 were added in a basic medium (Apel II medium) (hereinafter referred to as 'Step II'). The cells cultured up to Day 6 was termed pro hemogenic endothelial cells (pro HEs), and the hemogenic endothelial cells were subcultured on Day 6 to be isolated with high purity. Here, 0.25 % trypsin/EDTA was treated for 3 minutes in an incubator to confirm that non-hemogenic endothelial cells distributed as a single layer fell off first. Next, following washing with DPBS, 0.25 % trypsin/EDTA was additionally treated to isolate clusters of the hemogenic endothelial cells into single cells. After filtering the isolated single cells through a 0.44 µm filter, the cells from a single well were divided into 2 wells, and then subcultured for 21 days in a conditioned medium in which 11 ng/ml of bFGF, 50 ng/ml of VEGFA, 170 ng/ml of SCF, 150 ng/ml of FLT3L, 18 ng/ml of GCSF, 35 ng/ml of IL-6, 25 ng/ml of IGF-1, 15 ng/ml of IL-7, 10 ng/ml of IL-15, 25 ng/ml of IL-5, and 7 ng/ml of DLL1 were contained in a basic medium (Apel II medium) (hereinafter referred to as 'Step III'). A half medium change was maintained every 3 days to 4 days until Day 21.

### Preparation Examples 2 to 4.

The preparation of cells was carried out in the same manner as in Preparation Example 1, except that each step used configurations and contents shown in Tables 1 to 3.

**[Table 1]**

| | Composition | Preparation Example 2 | Preparation Example 3 | Preparation Example 4 | Preparation Example 5 |
|---|---|---|---|---|---|
| Step I | bFGF | 15 ng/ml | 9 ng/ml | 12 ng/ml | 17 ng/ml |
| | CHIR | 5 nM | 2nM | 4 nM | 7nM |
| | Ascorbic acid | 70 ng/ml | 80 ng/ml | 120 ng/ml | 150 ng/ml |
| | VEGFA | 18 ng/ml | 22 ng/ml | 25 ng/ml | 30 ng/ml |
| | SCF | 55 ng/ml | 45 ng/ml | 58 ng/ml | 65 ng/ml |
| | BMP4 | 20 ng/ml | 22 ng/ml | 30 ng/ml | 35 ng/ml |

**[Table 2]**

| | Composition | Preparation Example 2 | Preparation Example 3 | Preparation Example 4 | Preparation Example 5 |
|---|---|---|---|---|---|
| Step II | bFGF | 15 ng/ml | 9 ng/ml | 12 ng/ml | 17 ng/ml |
| | VEGFA | 18 ng/ml | 22 ng/ml | 25 ng/ml | 30 ng/ml |
| | SCF | 225 ng/ml | 195 ng/ml | 210 ng/ml | 270 ng/ml |
| | FLT3L | 170 ng/ml | 190 ng/ml | 210 ng/ml | 230 ng/ml |
| | GCSF | 25 ng/ml | 23 ng/ml | 27 ng/ml | 30 ng/ml |
| | TPO | 150 ng/ml | 120 ng/ml | 130 ng/ml | 180 ng/ml |
| | EPO | 23 ng/ml | 21 ng/ml | 25 ng/ml | 27 ng/ml |
| | IL-6 | 35 ng/ml | 45 ng/ml | 55 ng/ml | 65 ng/ml |
| | IGF-1 | 40 ng/ml | 35 ng/ml | 45 ng/ml | 55 ng/ml |

**[Table 3]**

| | Composition | Preparation Example 2 | Preparation Example 3 | Preparation Example 4 | Preparation Example 5 |
|---|---|---|---|---|---|
| Step III | bFGF | 13 ng/ml | 15 ng/ml | 20 ng/ml | 23 ng/ml |
| | VEGFA | 30 ng/ml | 70 ng/ml | 90 ng/ml | 110 ng/ml |
| | SCF | 230 ng/ml | 200 ng/ml | 300 ng/ml | 330 ng/ml |
| | FLT3L | 180 ng/ml | 210 ng/ml | 240 ng/ml | 270 ng/ml |
| | GCSF | 25 ng/ml | 22 ng/ml | 28 ng/ml | 32 ng/ml |
| | IL-6 | 45 ng/ml | 40 ng/ml | 55 ng/ml | 65 ng/ml |
| | IGF-1 | 40 ng/ml | 30 ng/ml | 50 ng/ml | 55 ng/ml |
| | IL-7 | 25 ng/ml | 18 ng/ml | 28 ng/ml | 35 ng/ml |
| | IL-15 | 23 ng/ml | 17 ng/ml | 30 ng/ml | 37 ng/ml |
| | IL-5 | 40 ng/ml | 30 ng/ml | 50 ng/ml | 65 ng/ml |
| | DDL1 | 35 ng/ml | 15 ng/ml | 28 ng/ml | 30 ng/ml |

### [Examples]

### Example 1. Characterization of hemogenic endothelial cells

The hemogenic endothelial cells cultured in Step III of Preparation Example 1 were divided into early hemogenic endothelial cells (EHE) and late hemogenic endothelial cells (LHE) based on Day 13 of the culture. Then, the characteristics of each cell were confirmed by using a microscope (x 100, x 200 and x 400).

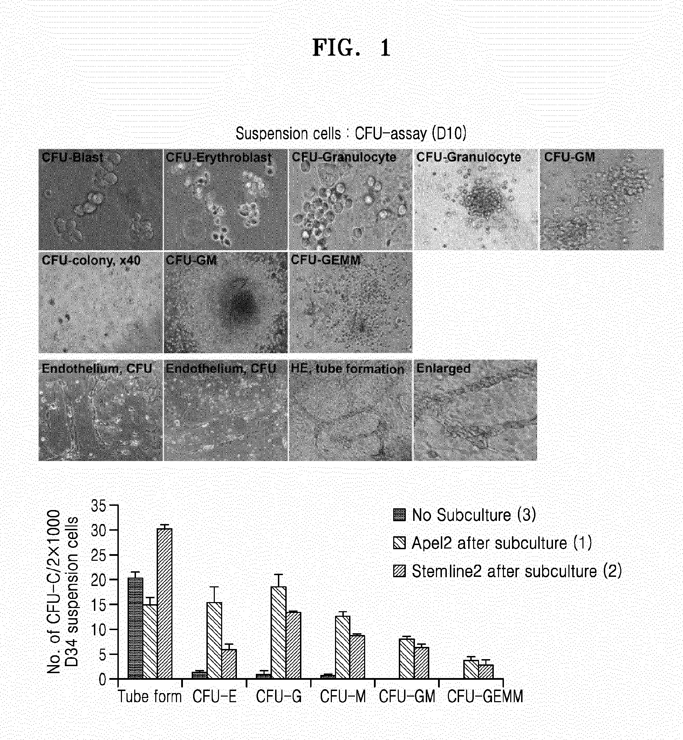

FIG. 1 shows the characteristics of early and late hemogenic endothelial cells.

As a result, as shown in FIG. 1, it was confirmed that the early hematopoietic endothelial cells formed clusters of cells that were not widely distributed it was confirmed that they formed clusters with a non-wide distribution of cells, and that other cells besides the early hemogenic endothelial cells were distributed together. Here, it was confirmed that marker proteins, e.g., CD31 and VE-cadherin, of the hemogenic endothelial cells were expressed.

In addition, in the case of the LHE cells after Day 13 of the culture, it was confirmed that more cells were aggregated compared to the EHE cells. Here, the emerging blood cells showed a definitive blood shape. It was confirmed that such LHE cells reached the end of lifespan as the cytoplasm became thinner and more tapered towards the latter period of the culture. In addition, it was confirmed that megakaryocyte-erythroid progenitors of FLK-1+ emerged from the LHE cells. From this single LHE, multiple megakaryocyte-erythroid progenitors were generated and subjected to clonal expansion, and as a result, it was confirmed that the hemogenic endothelial cells mainly showed a rod-like shape, which is distinct from the cobblestone-like shape of typical vascular endothelial cells or the shape of typical mesenchymal stromal cells. The blood cells generated by the LHE cells were mainly primitive blood cells (mainly myeloid lineage cells) in the early stage, and cells derived from the LHE cells included definitive blood cells (including both myeloid lineage cells and lymphoid lineage blood cells). Accordingly, tube formation in the resulting vascular endothelial cells was observed. Referring to the results above, it is considered that the hemogenic endothelial cells had characteristics of both vascular endothelial cells and blood cells.

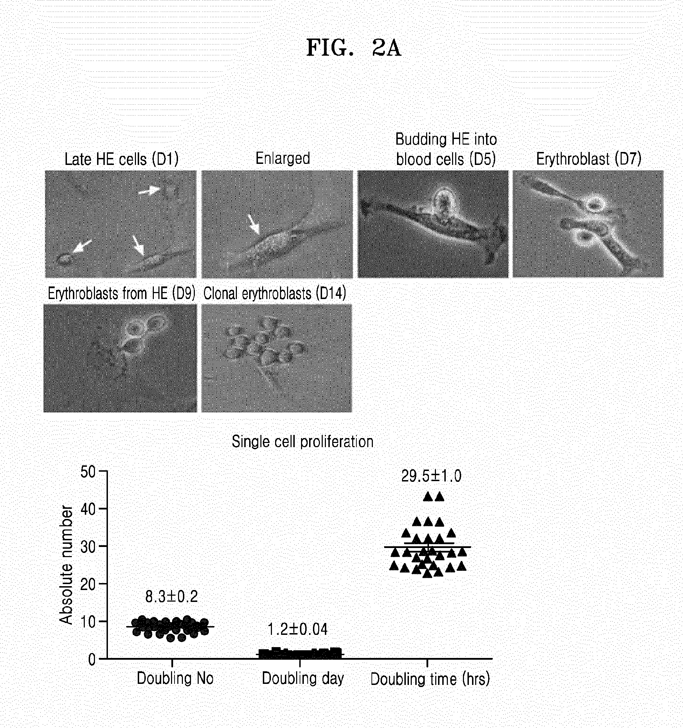

FIG. 2A shows photographs depicting the cell morphology of the LHE cells according to the culture time and shows a graph of proliferation of single cells.

As a result, as shown in FIG. 2A, bright yellow light was observed under a microscope on Day 1 of the culture of the LHE cells, due to bright light (indicated by white arrows) presumed to be caused by 5-aminolevulinate synthase which is a precursor of porphyrin. However, it was confirmed that the bright light disappeared from the hemogenic endothelial cells finished generating erythroblasts (on Day 9 and after Day 10). In addition, the shape of budding erythroblasts was observed in the hemogenic endothelial cells on Day 5, and the formation of erythroblasts was confirmed after Day 7. It was confirmed that the doubling day of the hemogenic endothelial cells was about 1.2 days and the average doubling time was about 29.5 hours.

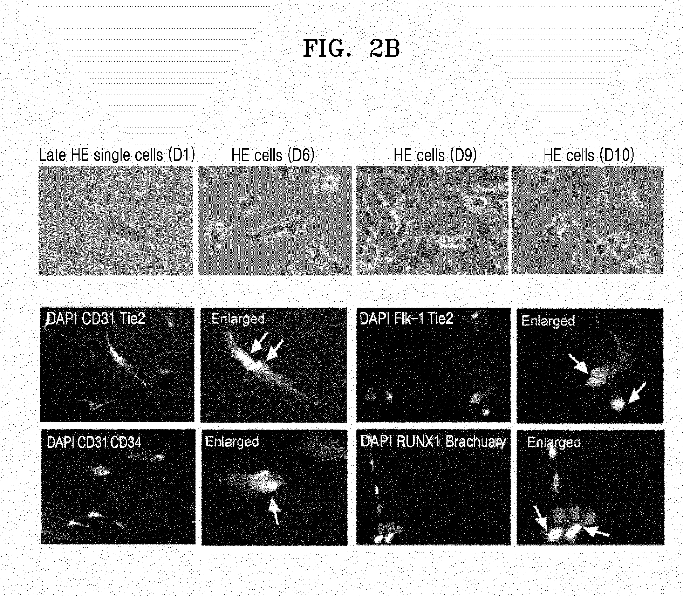

FIG. 2B shows photographs confirming expression of marker proteins in the LHE cells.

As a result, as shown in FIG. 2B, it was confirmed that marker proteins, such as CD31, Tie-2, CD34, etc., were expressed in the hemogenic endothelial cells constituting the clusters after Day 9, and that mesoderm markers, such as RUNX1 and brachuary, remained in the nucleus in some cells. In addition, it was confirmed that FLK-1 protein was intensively expressed in blood cells when the blood cells emerged from the hemogenic endothelial cells.

### Example 2. Proliferation and differentiation of THE cells according to culture conditions

The LHE cells cultured in Example 1 were stored in a freezer for 1 month and then thawed in a water bath at 37 °C. Then, to confirm whether a medium was suitable for proliferation and differentiation of the LHE cells, three basic medium (i.e., Apel II, EGM-2, and DMEM/F12 medium) were used. In the case of Apel II and DMEM/F12 media, 7 ng /ml of bFGF, 15 ng/ml of VEGFA, 180 ng/ml of SCF, 150 ng/ml of FLT3L, 18 ng/ml of GCSF, 80 ng/ml of TPO, 17 ng/ml of EPO, 25 ng/ml of IL-6, 30 ng/ml of IGF-1, 45 ng/ml of IL-3, and 1 nM of CHIR were added. In the case of EGM-2 medium, the same type and content of cytokines as in Step III of Preparation Example 1 were additionally added. Afterwards, the LHE cells thawed after freezing were cultured in each of the three aforementioned media, and all media were maintained with a half medium change once every 3 days to 4 days. Since the LHE cells are vascular endothelial cells thawed after freezing, the LHE cells were expected to attach to the culture dish and proliferate.

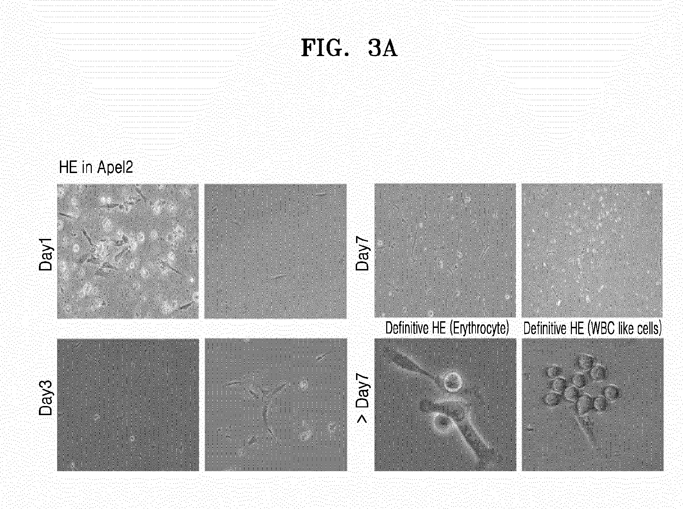

FIG. 3A shows photographs depicting the cell morphology on Day 1, Day 3, Day 7 and beyond, after culturing the hemogenic endothelial cells in the Apel II basic medium.

As a result, as shown in FIG. 3A, it was confirmed that most of the hemogenic endothelial cells failed to attach to the culture dish and rapidly died in the Apel II basic medium. However, in the case of the hemogenic endothelial cells that survived beyond Day 7, it was confirmed that WBC-like cells were generated or erythroblast colonies were formed when functions as endothelial cells were stabilized. However, these hemogenic endothelial cells showed significantly lower generation than generation of blood cells from the hemogenic endothelial cells cultured in the EGM-2 and DMEM/F12 basic media.

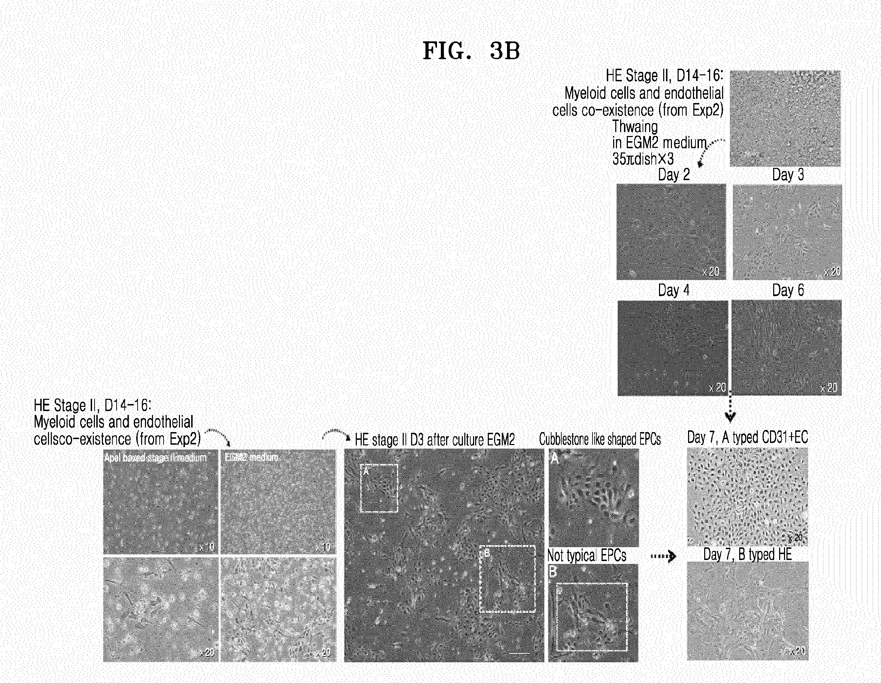

FIG. 3B shows photographs depicting the cell morphology on Day 1 and Day 3, after culturing the hemogenic endothelial cells in the EGM-2 basic medium.

As a result, as shown in FIG. 3B, the hemogenic endothelial cells cultured in the EGM-2 basic medium proliferated stably after being attached to the culture dish, and the proliferation rate thereof was also fast and stable compared to other basic media. In particular, in the case of the hemogenic endothelial cells proliferated for 7 days or more, it was confirmed that these hemogenic endothelial cells were divided into a typical cobblestone-like-shaped endothelial progenitor cell population and an atypical vascular endothelial cell population. Meanwhile, among the hemogenic endothelial cells cultured in the Apel II basic medium, those failed the cell adhesion were cultured in an EGM-2 medium for 3 days to induce proliferation of adherent cells. Then, as a result of culturing again in an Apel II medium, the hemogenic endothelial cells were found to proliferate normally. That is, the EGM-2 basic medium is positive for the proliferation and engraftment of the hemogenic endothelial cell, and can affect the generation of blood cells by stable adhesion and proliferation of the hemogenic endothelial cells in the culture dish.

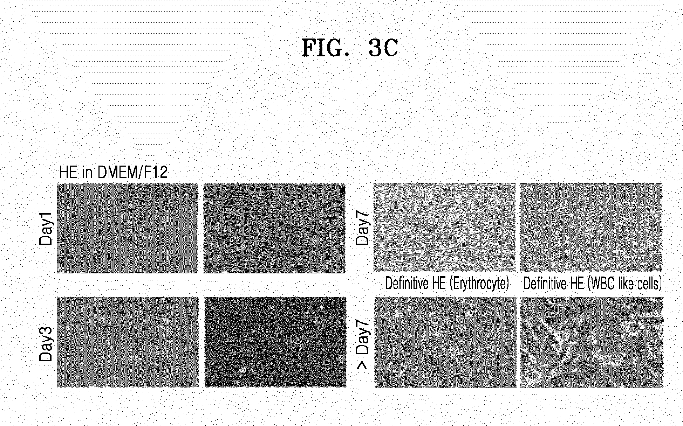

FIG. 3C shows photographs depicting the cell morphology on Day 1, Day 3, Day 7 and beyond, after culturing the hemogenic endothelial cells in the DMEM/F12 basic medium.

As a result, as shown in FIG. 3C, it was confirmed that the hemogenic endothelial cells cultured in the DMEM/F12 basic medium adhered to the culture dish and proliferated. Compared to the hemogenic endothelial cells cultured in the EGM-2 basic medium, the proliferation rate was 4 days to 5 days slower, but it was confirmed that many colonies of the hemogenic endothelial cells were formed so that large amounts of blood cells were stably generated after Day 8.

### Example 3. Identification of protein markers expressed in hemogenic endothelial cells

Expression of marker proteins expressed in the hemogenic endothelial cells depending on the basic media of Example 2 was compared. As a result, there was no significant difference in the expression of marker proteins depending on the basic media, and it was confirmed that typical marker proteins were expressed in the hemogenic endothelial cells.

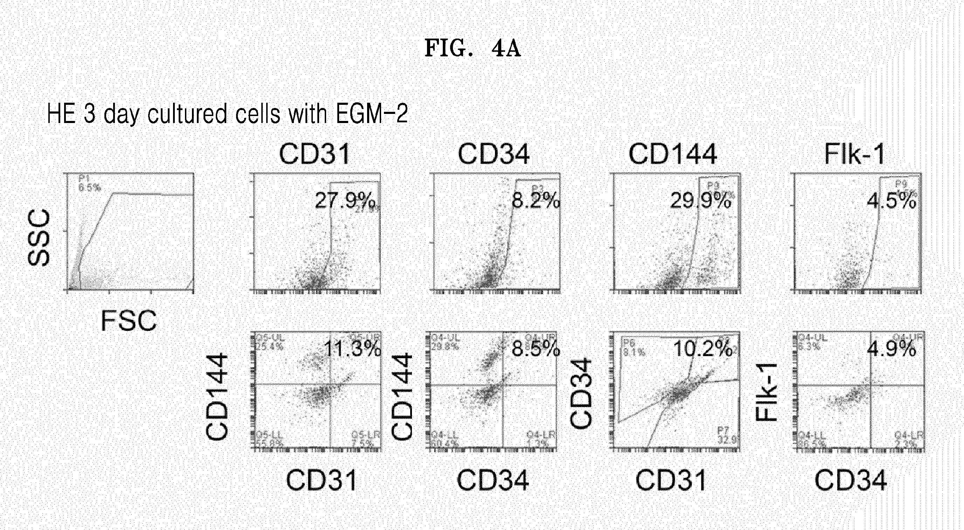

FIG. 4A shows a result of confirming the expression of marker proteins in the hemogenic endothelial cells cultured for 3 days in the EGM-2 basic medium.

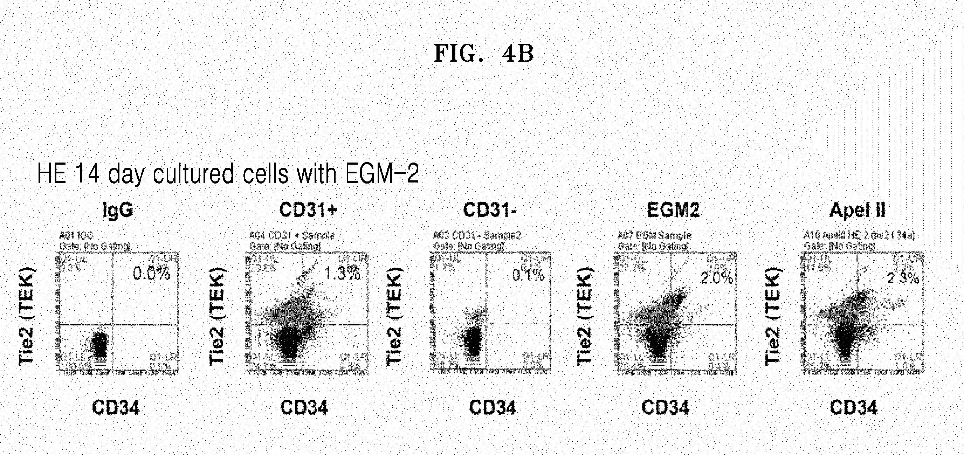

FIG. 4B shows photographs of confirming Tie-2 enrichment of the hemogenic endothelial cells cultured for 14 days in the EGM-2 basic medium.

As a result, as shown in FIG. 4a, it was confirmed that protein markers, such as CD31 27.9 %, CD34 8.2 %, CD144 29.9 %, and Flk-1 4.5 %, were expressed and a combination of protein markers, such as CD144+CD31+ 11.3 %, CD144+CD34+ 8.5 %, CD31+CD34+ 10.2 %, and Flk-1+CD34+ 4.9 %, were expressed, in a group of the hemogenic endothelial cells cultured in the EGM-2 basic medium. In addition, as shown in FIG. 4B, it was confirmed in an MACS-sorted group using CD31 that the expression of Tie-2 was enriched by 24.9 % in a CD31+ cell group and enriched by 1.8 % in a CD31- cell group. These results suggest the commitment of the CD31+ cells to the hemogenic endothelial cells. In addition, it was confirmed that the hemogenic endothelial cell markers were expressed in the order of Tie2 > CD144 > CD31 > CD34 > FLK1.

### Example 4. Confirmation of culture conditions for hemogenic endothelial cells to induce generation of lymphoid lineage blood cells

Based on the results of Example 2, culture conditions for the hemogenic endothelial cells to induce generation of lymphoid lineage blood cells were additionally confirmed. First, the aorta-gonad-mesonephros (AGM) and liver tissue were isolated from the fetus of mice 10.5 to 11.5 days old. Then, the tissue minced inside a cap of a 1.5 ml Eppendorf tube was washed with DPBS and then cultured in the same three media as in Example 2 until Day 20, the day of birth. To confirm the shape of the hemogenic endothelial cells cultured in each medium, hematoxylin & eosin (H&E) staining was performed.

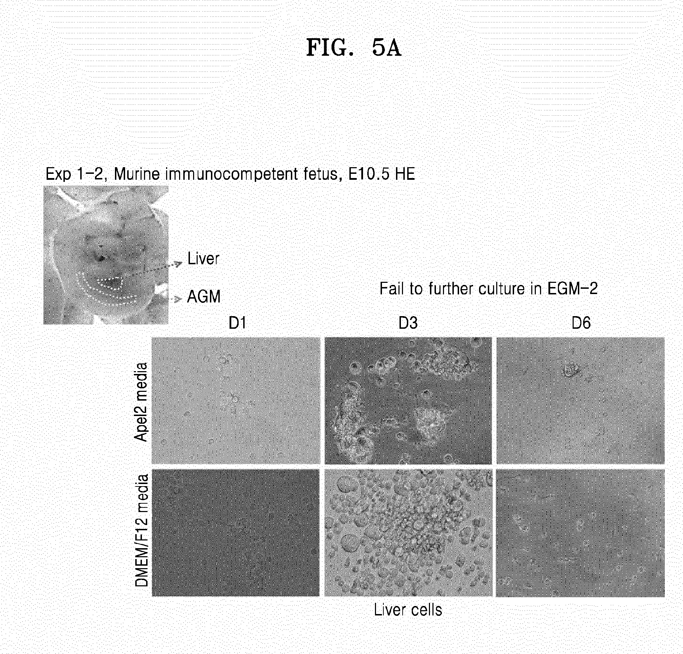

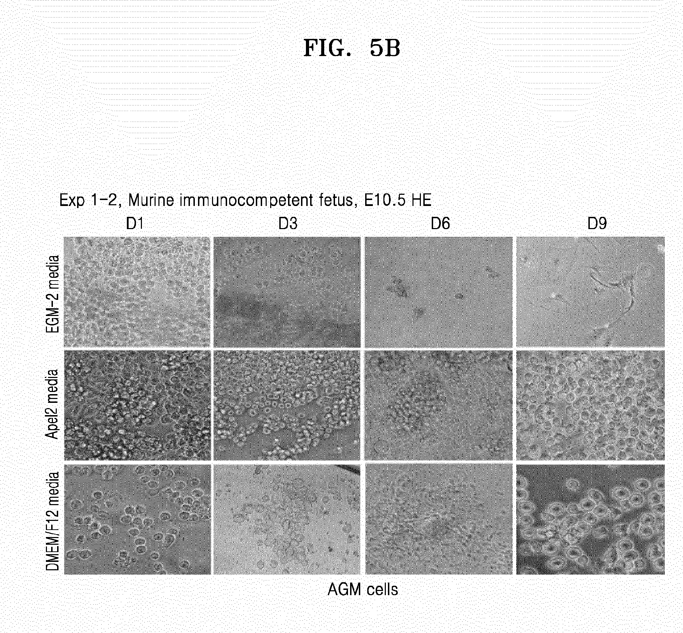

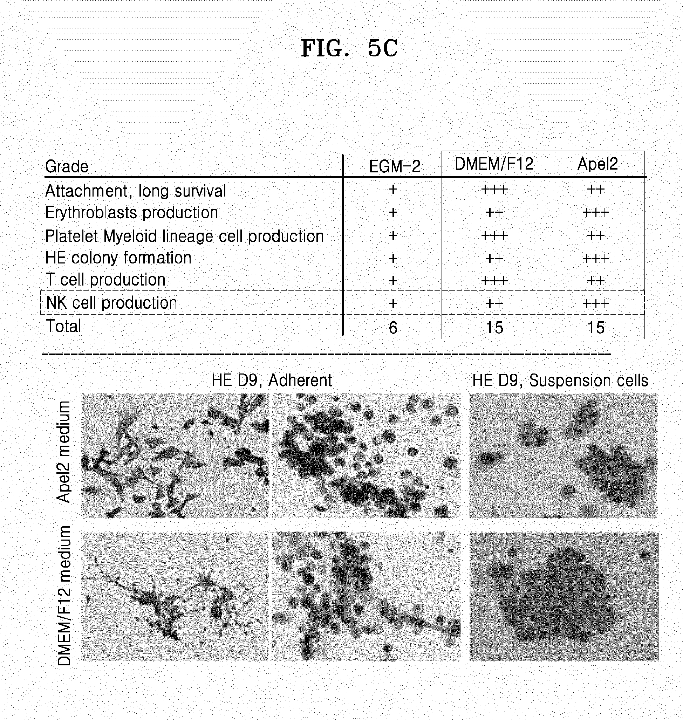

FIG. 5A shows results of confirming the generation of lymphoid lineage blood cells from the hemogenic endothelial cells in the liver of mice, and FIG. 5B shows results of confirming the generation of lymphoid lineage blood cells from the hemogenic endothelial cells in the AGM of mice. FIG. 5C shows a chart summarizing the properties and colony formation of the hemogenic endothelial cells cultured in each basic medium, and the degree of generation of lymphoid lineage blood cells and myeloid lineage cells, and also shows results of confirming the shape of the hemogenic endothelial cells.

Although acquisition of the hemogenic endothelial cells was rare in the liver tissue-derived blood cells, it was confirmed that the blood cells generated from the hemogenic endothelial cells showed different trends depending on the three media in the process of infiltration into the liver and the maturation process. Specifically, as shown in FIG. 5A, it was confirmed in the EGM-2 basic medium that all hemogenic endothelial cells died. On the other hand, it was confirmed in the Apel II basic medium that the hemogenic endothelial cells were rapidly induced to megakaryocyte-erythrocytes and pronormoblasts and even to orthochromic normoblasts within 3 days, and then, disappeared within 6 days. In addition, it was confirmed in the DMEM/F12 basic medium that differentiation into megakaryocyte-erythrocyte-derived megakaryoblasts and megakaryocytes (MK-II) was vigorous rather than into erythroid lineage cells, and the formation of proplatelet protrusions and silia was remarkably observed. Specifically, it was confirmed that more leukocytic lineage cells were generated than heme-bound erythroid lineage cells.

On the other hand, as shown in FIG. 5B, it was confirmed that the hemogenic endothelial cells present in the AGM differed in culture up to Day 9 depending on the culture medium. Specifically, it was confirmed in the EGM-2 basic medium that the disappearance of blood cells was remarkably increased. That is, unlike human-derived pluripotent hemogenic endothelial cells, it was confirmed that the mouse hemogenic endothelial cells did not survive for a long period of time. However, it was confirmed in the Apel II basic medium that the enrichment of erythroid lineage cells was rapidly achieved and the proliferation of leukocytic lineage cells was remarkably increased. In addition, it was confirmed in the DMEM/F12 basic medium that the degree of differentiation into megakaryocytes and leukocytes was significantly higher than the degree of differentiation into erythroid lineage cells. As a result, as shown in FIG. 5C, it was confirmed that the liver- and AGM-derived cells actively adhered and proliferated in the DMEM/F12 and Apel II basic media compared to the EGM-2 basic medium, enabling long-term differentiation into hemogenic endothelial cells and hemocytes.

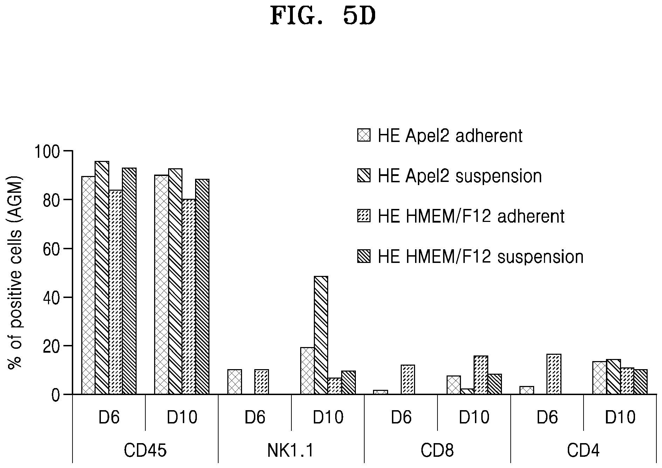

FIG. 5D shows a graph of results of analyzing expression levels of CD45, CD4, CD8, and NK1.1 by using differentiated cells in the AGM.

As a result, as shown in FIG. 5D, it was confirmed that the induction into natural killer cells within suspension cells was highest on Day 10 in the Apel II basic medium, and the induction into CD4 and CD8 cells along with megakaryocytes rapidly increased and maintained on Day 6 in the DMEM/F12 basic medium. Therefore, it was confirmed that the AGM-derived blood cells were maintained in both of the aforementioned two basic media.

### Example 5. Confirmation of differentiation of human pluripotent stem cells into hemogenic endothelial cells and blood cells

Based on the confirmation in Example 4 that the in vivo-derived hemogenic endothelial cells from the mouse fetus differentiated into blood cells, the LHE cells cultured in Example 1 were differentiated and proliferated in the medium of Example 2 to become blood cells, and then, surface markers were identified. Specifically, the hemogenic endothelial cells cultured for 21 days according to Steps I to III in Example 1 were additionally cultured until Day 34. Here, since the hemogenic endothelial cells were exfoliated or the frequency of apoptosis increases after Day 28 of the culture, the functional window of the hemogenic endothelial cells was set between Day 21 and Day 28. The hemogenic endothelial cells cultured until Day 34 were additionally treated with 0.25 % trypsin/EDTA to isolate single cells from cell clusters. Next, by performing CFU-assay, the formation of colonies of myeloid lineage cells was confirmed.

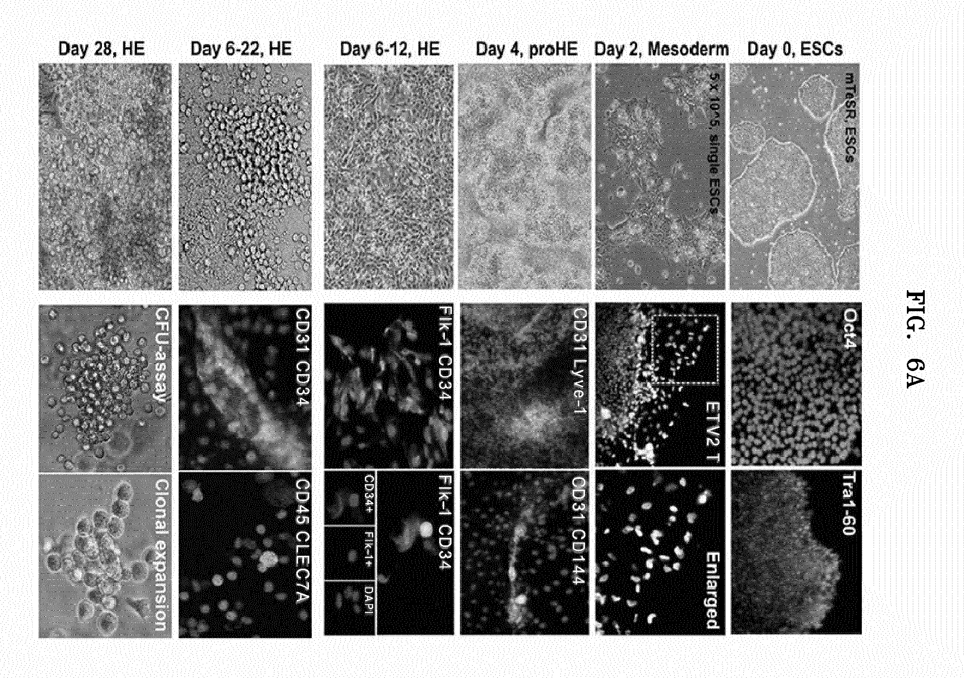

FIG. 6A shows results of the cell properties and expression markers at each generation stage of lymphoid lineage blood cells from the hemogenic endothelial cells derived from the human pluripotent stem cells.

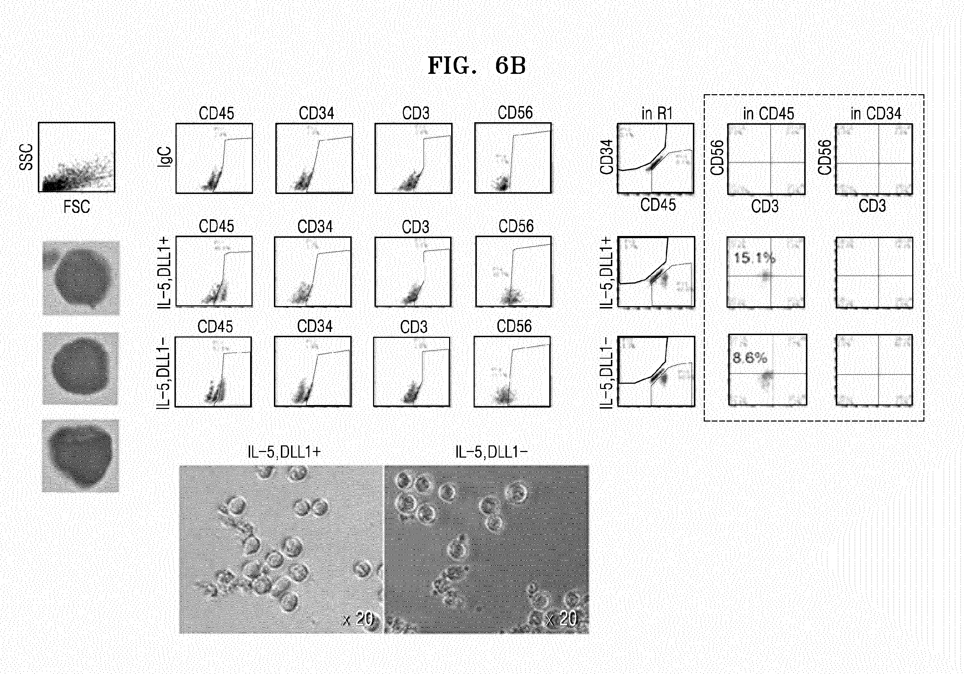

FIG. 6B shows a graph confirming, by expression of lymphoid lineage transcription factors, whether or not differentiation into lymphoid lineage blood cells is achieved in spots treated with IL-5 and DDL1.

As a result, as shown in FIG. 6A, it was confirmed that the hemogenic endothelial cells cultured from Day 13 to Day 34 formed colonies of the myeloid lineage cells and differentiated. In addition, as shown in FIG. 6B, it was confirmed that many lymphoid lineage transcription factors were expressed in spots treated with IL-5 and DLL1. That is, it was confirmed that the medium according to an aspect enables long-term culture of the human pluripotent stem cells, enabling differentiation into hemogenic endothelial cells and vascular cells (lymphoid lineage blood cells).

### Example 6. Confirmation of differentiation of human pluripotent stem cells into progenitor cells of T lymphocytes

Based on the confirmation in Example 4 that the in vivo-derived hemogenic endothelial cells from the mouse fetus differentiated into blood cells, the LHE cells cultured in Example 1 were differentiated and proliferated in the medium of Example 2 to become progenitor cells of T lymphocytes, i.e., γδT cells which are progenitor cells of αβT cells, and then, surface markers were identified. Specifically, the hemogenic endothelial cells cultured for 21 days according to Steps I to III in Example 1 were additionally cultured until Day 31. Here, since the generation of lymphoid cells is achieved through a definite hematopoiesis process on Day 31, the functional window of the hemogenic endothelial cells was set between Day 21 to Day 31. On Day 31 after the culture, FACS was performed to analyze cell surface markers.

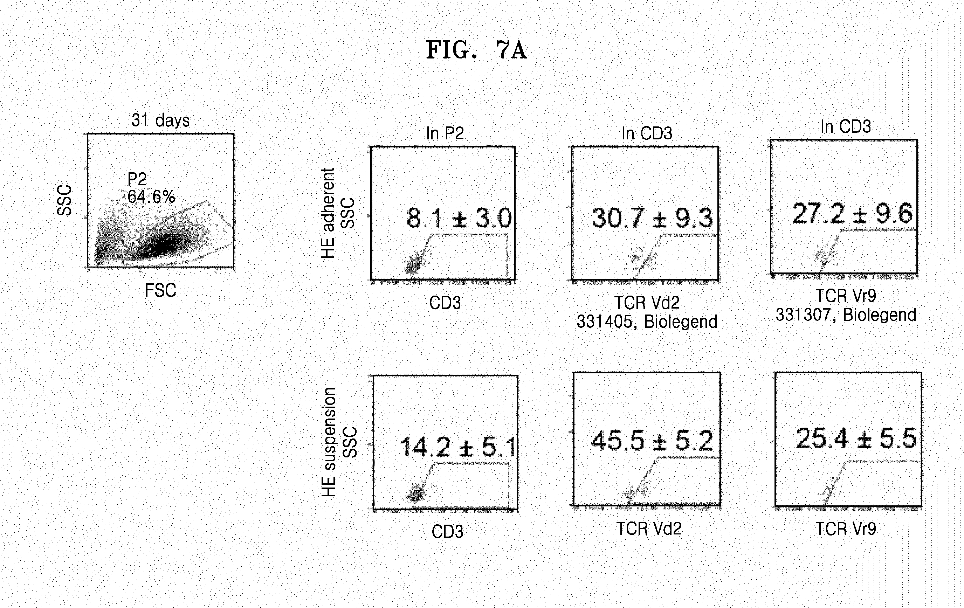

FIG. 7A shows results of confirming surface markers of γδT cells, which are progenitor cells of T lymphocyte, by FACS on Day 31 of the culture of the LHE cells derived from the human pluripotent stem cells.

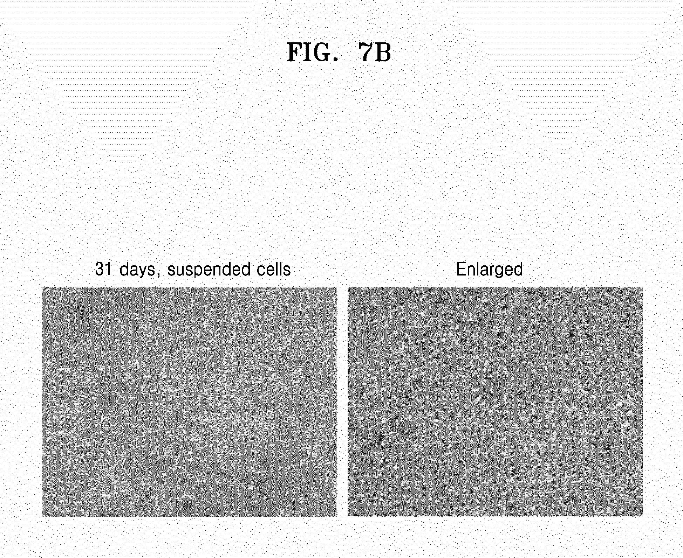

FIG. 7B shows graphs of quantification of surface markers of γδT cells, which are progenitor cells of T lymphocyte, on Day 31 of the culture of the LHE cells derived from the human pluripotent stem cells.

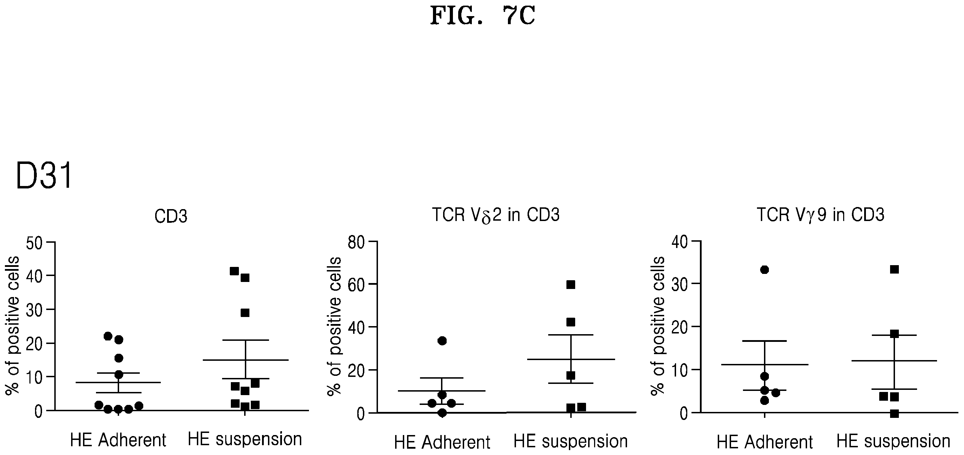

FIG. 7C shows results of confirming shape of γδT cells, which are progenitor cells of T lymphocyte, on Day 31 of the culture of the LHE cells derived from the human pluripotent stem cells.

As a result, as shown in FIGS. 7A and 7B, the LHE cells began to show expression of CD3 as being transitioned to the stage of definitive hemogenic cells on Day 22 of the culture. In addition, it was confirmed that the number of CD3+ cells was significantly high in suspension cells compared to adherent cells on Day 31 of the culture, with a frequency rate of about 40 %. In addition, TCR receptor subtypes could be identified in the CD3+ cells, and it was confirmed that expression of TCR Vδ2 was significantly high in suspension cells compared to adherent cells.

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A method of preparing a lymphoid lineage blood cell from a pluripotent stem cell, the method comprising:
differentiating a pluripotent stem cell into a mesodermal cell by culturing in a first medium containing a medium composition containing bFGF, VEGF, and SCF;
differentiating the mesodermal cell into an early hemogenic endothelial (EHE) cell by culturing in a second medium containing a medium composition containing TPO, EPO, and IGF-1; and
differentiating the EHE cell into a late hemogenic endothelial (LHE) cell by culturing in a third medium containing a medium composition containing IL-5, IL-7, and DLL.

2. The method of claim 1, wherein the pluripotent stem cell is selected from the group consisting of an embryonic stem cell, an induced pluripotent stem cell (iPSC), a somatic cell nuclear transfer-derived stem cell, and an adult-derived mesenchymal stem cell.

3. The method of claim 1, wherein the first medium further contains ascorbic acid, BMP4, or a mixture thereof.

4. The method of claim 1, wherein the second medium further contains: bFGF, VEGFA, SCF, FLT3L, GCSF, and IL-6; or a mixture of the aforementioned factors.

5. The method of claim 1, wherein the EHE cell has one or more characteristics selected from the following (a) to (d):
(a) having rod-type morphological characteristics;
(b) having bright yellow light;
(c) having a doubling time of 15 hours to 35 hours; and
(d) having surface antigen characteristics for CD31+, Tie-2+, CD144+, CD34+, or a combination thereof.

6. The method of claim 1, wherein the third medium further contains: bFGF, VEGFA, SCF, FLT3L, GCSF, and IL-6, IGF-1, IL15; or a mixture of the aforementioned factors.

7. The method of claim 1, wherein the LHE cell has one or more characteristics selected from the following (a) and (b):
(a) having cobblestone-like type morphological characteristics; and
(b) having surface antigen characteristics for CD31+, CD34+, CD144+, Flk-1+, CD144+CD31+, CD144+CD34+, CD31+CD34+, Flk-1+CD34+, or a combination thereof.

8. The method according to claim 1, wherein the differentiating of the pluripotent stem cell into the mesodermal cell is performed for 1 day to 3 days.

9. The method according to claim 1, wherein the differentiating of the mesodermal cell into the EHE cell is performed for 6 days to 8 days.

10. The method of claim 1, wherein the differentiating of the EHE cell into the LHE cell is performed for 12 days to 13 days.

11. The method of claim 1, further comprising differentiating the LHE cell into a lymphoid lineage blood cell by additionally culturing for 13 days to 28 days.

12. The method of claim 11, wherein the LHE cell is cultured in an EGM-2 basic medium containing at least one selected from the group consisting of bFGF, VEGFA, SCF, FLT3L, GCSF, IL-6, IGF-1, IL-7, IL-15, IL-5, and DLL1.
